# EUROPÄISCHE PATENTANMELDUNG

(11) **EP 3 747 406 A1**
(43) Veröffentlichungstag der Anmeldung: **09.12.2020**
(21) Anmeldenummer: 19178007.1
(22) Anmeldetag: 04.06.2019
(51) Int. Cl.: A61F 2/54, A61F 2/58, A61F 2/68

(54) **PROTHESE ZUM KLETTERN UND/ODER BOULDERN**

(71) Anmelder: ETH Zurich, 8092 Zurich (CH)
(72) Erfinder: TROJAN, Andreas, 8032 Zürich (CH); ANTOSCH, Alexander, 8052 Zürich (CH); MARTI, Hannah, 8765 Engi (CH); SCHOLLENBERGER, Fabian, 8127 Forch (CH)

(57) **Zusammenfassung**

Es soll eine Prothese (0), umfassend einen Prothesenschaft (1) an dessen distalen Ende ein proximales Ende einer Kupplung (2) mit einer verbundenen Exoprothese (3) angeordnet ist, wobei der Prothesenschaft (1) eine einschnürbare Aussenschale (11) mit einer Schalenaussenfläche (111) umfasst und eine Verschlusseinrichtung (112) mit mindestens einem Spannmittel (1121), Halte- und Umlenkmitteln (1122) und mindestens ein Verschluss (1123) flächig auf der Schalenaussenfläche (111) verteilt angeordnet ist, für den Einsatz beim Klettern oder Bouldern optimiert werden. Dies wird dadurch erreicht, dass der Prothesenschaft (1) mindestens annähernd linear in Richtung Längsachse (L) relativ zur Exoprothese (3) einen Spalt (Δl) zwischen Prothesenschaft (1) und Exoprothese (3) ausbildend verschiebbar an der Exoprothese (3) oder verschiebbar an einer Kupplung (2) zwischen Prothesenschaft (1) und Exoprothese (3) lösbar befestigbar ist.

## Beschreibung

### Technisches Gebiet

Die vorliegende Erfindung beschreibt eine Prothese, umfassend einen Prothesenschaft an dessen distalen Ende ein proximales Ende einer Kupplung mit einer verbundenen Exoprothese angeordnet ist, wobei der Prothesenschaft eine einschnürbare Aussenschale mit einer Schalenaussenfläche umfasst und eine Verschlusseinrichtung mit mindestens einem Spannmittel, Halte- und Umlenkmitteln und mindestens ein Verschluss flächig auf der Schalenaussenfläche verteilt angeordnet ist, sowie eine Kupplung einer Prothese, zur Ankupplung einer Exoprothese an einen Prothesenschaft, wobei der Prothesenschaft eine Aussenschale mit einer Schalenaussenfläche aufweist, auf welcher flächig verteilt eine Verschlusseinrichtung mit mindestens einem Spannmittel, mehreren Halte- und Umlenkmitteln und einem Verschluss angeordnet ist.

### Stand der Technik

Es sind verschiedene Prothesen für die oberen Extremitäten von Menschen seit langem bekannt. Heute umfassen derartige Prothesen in der Regel einen Prothesenschaft, eine Kupplung und eine Expoprothese als Aufsatz. Der Prothesenschaft ist ein Modul der Prothese, welches die Verbindung zwischen dem mechanischen Ersatz der Extremität und dem verbliebenen Extremitätenstumpf bildet.

Der Extremitätenstumpf wird in das proximalen Ende des Prothesenschafts, das dem Prothesenträger zugewandte Ende, eingeführt und darin befestigt, wofür es verschiedene Techniken gibt. Der Prothesenschaft soll möglichst eng und formschlüssig auf dem Extremitätenstumpf sitzen, damit eine ungewünschte Lockerung vermieden wird. Der Tragekomfort wurde in den letzten Jahren durch die Optimierung der passgenauen Verbindung zwischen dem Extremitätenstumpf und dem Prothesenschaft enorm verbessert. Der Prothesenschaft umfasst in der Regel einen inneren elastischen Prothesenschuh, der flexibel auf der Haut des Trägers aufliegt, um Druckstellen zu vermeiden. Um den inneren Prothesenschuh wird meist eine Schaumlage und eine starre Aussenschale gelegt, welche möglichst steif ausgeführt ist. Die starre Aussenschale nimmt Kräfte auf, welche nur teilweise an den inneren Prothesenschuh weitergeleitet werden. Ist ein Prothesenschaft erstmal an einen Extremitätenstumpf angepasst, dann kann die Prothese über einen längeren Zeitraum verwendet werden.

Um die Anpassung der Aussenschale des Prothesenschafts an den Extremitätenstumpf zu vereinfachen und die Festigkeit zu verbessern hat die US2004/158332 einen Weg gezeigt. Darin wird erläutert, dass der Prothesenschaft einen inneren Prothesenschuh aufweist, welcher samt Extremitätenstumpf in eine einstellbare starre Aussenschale geführt wird, sodass eine verbesserte Fixierung erreicht wird. Der Prothesenschaft weist Längsschlitze auf und eine Verschlusseinrichtung mit zwei umlaufenden Bändern und Spannverschlüssen. Die Bänder umlaufen den Prothesenschaft auf unterschiedlichen Höhen und können, wie von Skischuhen bekannt, mit den Spannverschlüssen festgestellt werden, wodurch der Prothesenschaft mit dem Extremitätenstumpf verbunden wird. Durch die Verschlusseinrichtung wird der Durchmesser des Prothesenschafts verringert und auf dem Extremitätenstumpf fixiert.

Die Kupplung für die Exoprothese, welche in unterschiedlichen Gestaltung als Aufsatz montiert wird, wird mit einem Klemmring mittels Schrauben indirekt am Prothesenschaft, über einen Bolzen, der an einem inneren schlauchförmigen Prothesenschuh aus Silikon befestigt ist, verschraubt. Der Prothesenschaft ist somit stärker am Extremitätenstumpf befestigt, wobei die Kupplung einfach montier- und demontierbar bleibt. Die Verschlusseinrichtung an der Aussenfläche des Prothesenschafts ist aber sehr klobig ausgeführt, was bei Prothesen für die oberen Extremitäten unerwünscht ist.

Die US020140005798 beschreibt unter anderem auch Prothesen für obere Extremitäten, wobei eine einfachere und leichtere Verschlusseinrichtung auf einer Schalenaussenfläche des Prothesenschafts angeordnet ist. Diese Verschlusseinrichtung ist flaschenzugartig ausgeführt und umfasst Spannmittel, Halte- und Umlenkmittel und mindestens einen Verschluss.

An den Prothesenschaft der US020140005798 könnte eine Exoprothese in Form eines Hakens zum Klettern oder Bouldern montiert werden. Bei der Benutzung einer solchen Prothese ist der Tragekomfort und der Eindruck beim Klettern oder Bouldern aber nicht annähernd natürlich. Da dabei grosse Zugkräfte wirken, die direkt vom Prothesenschaft aufgenommen werden, müsste die Verschlusseinrichtung enorm straff gezogen sein, damit der Extremitätenstumpf nicht aus dem Prothesenschaft bzw. einem inneren Prothesenschuh herausrutscht. Der Prothesenträger hat das Gefühl, dass die Zugkräfte auf den gesamten Extremitätenstumpf wirken, wobei der Extremitätenstumpf zusätzlich noch vom Prothesenschaft gequetscht wird.

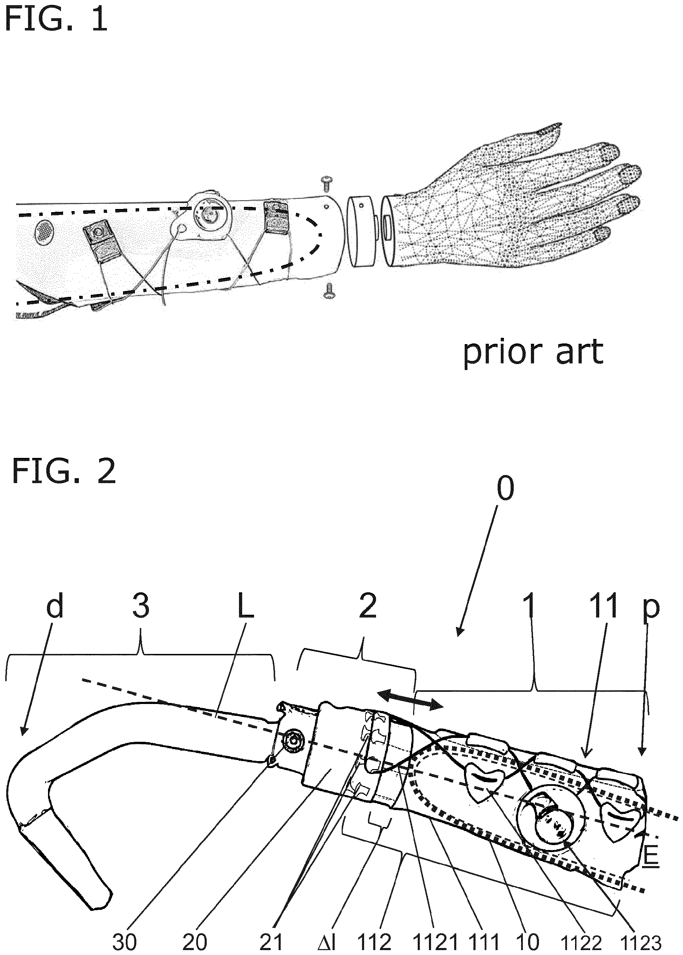

Auch in der WO2018054966 ist eine Verschlusseinrichtung ausschliesslich auf einer Schalenaussenfläche der Aussenschale des Prothesenschafts vorgeschlagen. Eine entsprechende Prothese ist in Figur 1 als Beispiel für den Stand der Technik gezeigt. Durch die Verschlusseinrichtung mit zugehörigem Verschluss, Spannmitteln und Halte- und Umlenkmitteln entlang der Schalenaussenfläche der Aussenschale, wird der Extremitätenstumpf optimal variierbar gehalten und umschlossen. Da eine Schaumstofflage zwischen Aussenschale und Prothesenschuh angeordnet ist, werden Druckstellen vermieden und Kräfte auf eine grössere Fläche verteilt. Die Spannmittel verlaufen vom proximalen bis zu einem distalen Ende des Prothesenschafts, welches dem Extremitätenstumpf bzw. dem Prothesenträger abgewandt ist, um Kräfte auf den gesamten Prothesenschaft zu verteilen.

Die Exoprothese ist mittels einer Kupplung am distalen Ende des Prothesenschafts befestigbar. Prothesenschaft, Kupplung und Exoprothese bilden im Benutzungszustand eine Einheit, wobei es keine lineare Relativbewegung zwischen Prothesenschaft, Kupplung und Exoprothese gibt und die Prothese als einstückige Einheit wirkt. Möchte der Träger andere Funktionen seiner Prothese erreichen, reicht es aus, die Exoprothese an der Kupplung zwischen Aussenschale und Exoprothese zu wechseln, wobei der Prothesenschaft und die Kupplung unverändert bleibt. Durch die Wahl unterschiedlicher Ausgestaltungen der Exoprothese als Aufsatz, kann der Träger heute dadurch viele Handfunktionen nachbilden.

An Prothesen mit derart sicher fixierbaren Prothesenschäften und daran fix befestigten Kupplungen, können einige unterschiedlich ausgeführte Exoprothesen einfach und schnell befestigt werden.

Der Prothesenschaft kann durch Betätigung der Verschlusseinrichtung derart manipuliert werden, dass der Umfang der Aussenschale variiert wird. Dadurch wird der Sitz der Aussenschalen und des inneren Prothesenschuhs angepasst. Egal, ob der Extremitätenstumpf breiter (in medio-lateraler Richtung) oder dünner (in anterior-posteriorer Richtung) wird, der Sitz des Prothesenschafts und damit der Prothese kann immer optimal eingestellt werden. Diese Einstellung an der Verschlusseinrichtung kann bevorzugt mit einer Hand erfolgen.

Eine Prothese, die dem Prothesenträger das Klettern oder Bouldern ermöglicht, wobei der Sitz und der Tragekomfort selbst bei extremen Belastungen und Zugkraftwirkungen der Prothese verbessert ist und einen natürlichen Eindruck liefert, ist aber mit diesen aus dem Stand der Technik bekannten Prothesen unerreicht. Da Prothesenträger auch Trendsportarten ausüben möchten, ist der Bedarf für derartige Prothesen, die einen natürlichen Eindruck bei der Ausübung des Sports liefern, vorhanden.

### Darstellung der Erfindung

Die oben beschriebenen aus dem Stand der Technik bekannten Nachteile, sollen durch die vorliegende Vorrichtung beseitigt werden.

Die vorliegende Erfindung hat sich zur Aufgabe gestellt eine Prothese für die oberen Extremitäten zu schaffen, welche bevorzugt zum Klettern und/oder Bouldern einsetzbar ist. Mit der Prothese sollen höhere Zugkräfte aufnehmbar sein. Insgesamt soll der Tragekomfort stark verbessert werden und der Prothesenträger in die Lage versetzt werden, einer sportlichen Betätigung, bevorzugt im Bereich Klettern und Bouldern für längere Zeiträume nachzugehen. Trotz wenig Gewicht soll eine robuste Prothese geschaffen werden, welche dauerhaft an einem Extremitätenstumpf befestigbar ist und dem Träger die Sicherheit gibt, dass der Prothesenschaft nicht vom Extremitätenstumpf abrutscht. Aufgrund der Gestaltung der Prothese erhält der Prothesenträger einen realistischeren Eindruck beim Klettern oder Bouldern.

Diese Aufgabe wird mit einer Prothese gemäss Anspruch 1 erreicht. Zur Lösung der Aufgabe wird die Verschlusseinrichtung samt Spannmitteln von der Aussenschale über die Kupplung verteilt und so Zugkräfte in neuartiger Art und Weise entlang der Prothese verteilt.

Die gestellte Aufgabe wird durch Einführung einer neuen Kupplung gemäss Anspruch 9 erreicht.

Alle möglichen Variationen von Merkmalskombinationen bzw. geringfügige Anpassungen der Erfindung, welche zu unterschiedlichen technischen Effekten führen können, sind in der Detailbeschreibung zu finden, in den Figuren abgebildet und in die abhängigen

Patentansprüche aufgenommen worden.

### Kurze Beschreibung der Zeichnungen

Der Erfindungsgegenstand wird nachstehend detailliert im Zusammenhang mit den anliegenden Zeichnungen beschrieben. Notwendige Merkmale, Einzelheiten und Vorzüge der Erfindung ergeben sich aus der nachfolgenden Beschreibung, wobei mindestens eine bevorzugte Ausführungsform der Erfindung und einige Zusatzmerkmale im Detail aufgeführt sind.
- Figur 1: zeigt eine perspektivische Explosionsdarstellung einer dreiteiligen Prothese gemäss Stand der Technik.
- Figur 2: zeigt eine perspektivische Darstellung einer erfindungsgemässen Prothese in einem ausgelenkten Zustand, während
- Figur 3a: eine schematische Darstellung einer Prothese im Grundzustand und
- Figur 3b: eine schematische Darstellung einer Prothese im ausgelenkten Zustand zeigt.
- Figur 4: zeigt eine schematische Schnittansicht einer Prothese, wobei die sonst als Kletterwerkzeug ausgebildete Exoprothese hier weggelassen wurde und ein ausgelenkter Zustand eines an der Kupplung montierten Prothesenschafts gezeigt ist.
- Figur 5: zeigte eine schematisch Schnittansicht einer Prothese, wobei die Kupplung mit einer Führung und der Prothesenschaft hülsenförmig gestaltet sind.
- Figur 6: zeigt eine schematisch Schnittansicht einer Prothese, wobei die Kupplung in die Exoprothese integriert ist und der Spalt zwischen Prothesenschaft und Exoprothese ausbildbar ist.
- Figur 7: zeigt eine weitere schematische Schnittansicht einer Prothese, wobei die Kupplung oder die Exoprothese mit dem Prothesenschaft über das mindestens eine Spannmittel wirkverbunden sind.

### Beschreibung

Es wird eine Prothese 0 vorgestellt, welche einen Prothesenschaft 1, eine Kupplung 2 und einem Tool 3 umfasst. Der Prothesenschaft 1 kann an einem Extremitätenstumpf E befestigt werden und umfasst im Inneren einen schlauchförmigen Prothesenschuh 10. Hier ist der Prothesenschaft 1 am Beispiel eines Unterarmprothesenschaftes 1 für eine Exoprothese 3 in Form einer Handprothese gezeigt. Derartige Prothesenschäfte 1 werden auch transversale Prothesenschäfte 1 genannt, da eine Einschnürung um den Extremitätenstumpf E erfolgen kann. Die Exoprothese 3 kann mittels der Kupplung 2, welche starr und robust ausgeführt ist, am Prothesenschaft 1 befestigt werden. Die Exoprothese 3 kann aber auch ohne Kupplung 2 indirekt am Prothesenschaft 1 befestigt werden.

Der Prothesenschaft 1 wird hier als zweistückig definiert, wobei der Prothesenschaft 1 mit der Kupplung 2, bei deren Vorhandensein, eine Einheit bildet, aber trotzdem eine lineare Relativbewegung zwischen Kupplung 2 und damit der Exoprothese 3 und dem Prothesenschaft 1 in Richtung der Längsachse L möglich ist. Damit kann diese Prothese 0 einen Grundzustand und ausgelenkte Zustände annehmen, von denen einer in Figur 2 gezeigt ist.

Der schlauchförmige Prothesenschuh 10 ist flexibel und bevorzugt mehrlagig ausgeführt. Insbesondere kann der Prothesenschuh 10 eine Hautauflage und eine flexible Schafthülllage aufweisen, welche durch Verbindungsnähte verbunden sind. Diese Details sind hier nicht in den Figuren dargestellt, da sie in verschiedenen Varianten ausführbar sind und nicht entscheidend für die vorliegende Erfindung. Die Verbindungsnähte werden bevorzugt durch Schweissung und Klebung erstellt, sodass ein flexibler doppelwandiger Prothesenschuh 10 erreicht wird.

Die Hautauflage sollte aus einem elastischen Material hergestellt sein, welches hautverträglich sein sollte, da es den Extremitätenstumpf E direkt umschliessend direkt auf der Haut aufliegt. Beispielsweise kann eine Lage Orthopädiegummi als Hautauflage fungieren. Es kann ein Orthopädiegummi oder Zellgummi mit einer Dicke von 3mm verwendet werden, welches geschlossenzellig ausgeführt ist. Dann sind Löcher eingebracht, die eine Lüftung erlauben. Wenn ein offenzelliger Elastomerschaumstoff verwendet wird, ist die Hautauflage atmungsaktiv, wobei Feuchtigkeit zum Textilgewebe, also weg von der Haut im Gebrauch diffundieren kann. Die Hautauflage kann aber auch aus einem Thermoplast oder Silikon hergestellt sein. Durch die Elastizität der Hautauflage passt sich diese den Körperkonturen, also der Oberfläche des Extremitätenstumpfes E optimal an. Durch die Rutschfestigkeit der Elastomerschaumstoffschicht, kann eine Vorfixierung der Hautauflage nach Überzug des schlauchförmigen Prothesenschuhs 10 erreicht werden. Der schlauchförmige Prothesenschuh 10 wird von distal d in Richtung proximal p über den Extremitätenstumpf E samt dem Prothesenschaft 1 gezogen. Eine Anpassung des Prothesenschuhs 10 und des Prothesenschaft 1 an den Extremitätenstumpf E ist meist einmalig vorgängig der ersten Benutzung der Prothese 0 nötig.

Der Prothesenschaft 1 wird neben dem schlauchförmigen Prothesenschuh 10 von einer Aussenschale 11 mit einer Schalenaussenfläche 111, auf welcher eine Verschlusseinrichtung 112 angeordnet ist und der Kupplung 2 gebildet und ist darum mehrstückig, hier zweistückig. Es ist trotzdem eine Einheit aus Prothesenschaft 1 und Kupplung 2 erreicht. Die Aussenschale 11 umgibt den Prothesenschuh 10 im einsatzbereiten Zustand der Prothese 0. Die Aussenschale 11 ist einschnürbar ausgestaltet, sodass der Umfang der Aussenschale 11 in Richtung Prothesenschuh 10 und damit Extremitätenstumpf E verringert werden kann. Eine derartige einschnürbare Aussenschale 11 wird auch als transradiale Aussenschale 11 bezeichnet, welche eine Radiusverringerung in Richtung Extremitätenstumpf E erlaubt.

Die Verschlusseinrichtung 112 ist in Figur 2 schematisch gezeigt und mindestens teilweise auf der Schalenaussenfläche 111 gelagert, wobei die Teile der Verschlusseinrichtung 112 die Schalenaussenfläche 111 mindestens teilweise umgeben.

Die Verschlusseinrichtung 112 umfasst hier mindestens ein Spannmittel 1121 in Form mindestens eines Seils 1121, welches zwischen einem Verschluss 1123 und einer Mehrzahl von Halte- und Umlenkmitteln 1122 gespannt werden kann. Die Verschlusseinrichtung 112 bzw. der Verschluss 1123 können unter anderem in Form des "Boa Closure Binding Systems" gewählt sein, welches kommerziell erhältlich ist.

Die Verschlusseinrichtung 112 hat die Aufgabe die Aussenschale 11, welche aus mehreren Teilen zusammengesetzt oder mit Längsschlitzen versehen ausgestaltet ist, zu umfassen und je nach Spannzustand unterschiedlich stark um den Prothesenschuh 10 zu spannen und damit am Extremitätenstumpf E zu befestigen. Die Aussenschale 11 kann auch aus mehreren Schalenteilen zusammengesetzt sein. Wird die Verschlusseinrichtung 112 bzw. der Verschluss 1123 betätigt, kann das mindestens eine Seil 1121 gespannt oder entspannt werden, wodurch der Durchmesser der Aussenschale 11 variierbar ist.

Damit gewährleistet die Verschlusseinrichtung 112 die Formgebung der Aussenschale 11 und die Fixierung der Aussenschale 11 am schlauchförmigen üblicherweise mehrwandigen Prothesenschuh 10. Die Aussenschale 11 muss derart formstabil ausgebildet werden, dass zum einen die Verschlusseinrichtung 112 gehalten werden kann und der schlauchförmige Prothesenschuh 10 am Extremitätenstumpf E gehalten wird, sodass der Prothesenschuh 10 vor Stössen geschützt wird und ein Abrutschen der Aussenschale 11 vom Extremitätenstumpf E verhindert ist.

Mittels Verschlusseinrichtung 112, wird die Aussenschale 11 den Prothesenschuh 10 umgebend gehalten. Vom Verschluss 1123 ausgehende Seile 1121 als Spannmittel 1121 sind um die Aussenschale 11 gelegt und durch Haken 1122 als Halte- und Umlenkmittel 1122 geführt. Die Haken 1122 sind auf der Schalenaussenfläche 111 verteilt und können unterschiedliche Formen annehmen. Dabei lenken die Haken 1122 die Spannmittel 1121 auf verschiedenen Bahnen um die Aussenschale 11. Dabei sind die Haken 1122 derart gestaltet, dass die auftretenden Zugkräfte aufgenommen werden können, ohne, dass die Haken 1122, das mindestens eine Seil 1121 oder die Schalenaussenfläche 111 Schaden nimmt.

Die Verschlusseinrichtung 112 ist als Seilzugvorrichtung mit entsprechenden Seilen 1121 und den Haken 1122 ausgestaltet, wobei der Einsatz anders gestalteter Umlenkmittel 1122 möglich ist. Durch Drehen des Verschlusses 1123 kann die Spannung der Seile 1121 variiert werden, womit der Sitz der Aussenschale 11 auf dem Prothesenschuh 10 und damit auf dem Extremitätenstumpf E variiert werden kann.

Mit der hier beschriebenen Verschlusseinrichtung 112 wird eine Aussenschale 11 und eine Justiermöglichkeit der Aussenschale 11 erreicht, sodass ein schmerzfreies fassen des Extremitätenstumpfes E resultiert. Trotzdem werden lokale Druckstellen verhindert. Das Anziehen und Ausziehen des Prothesenschafts 1 ist mit einer Hand möglich und während der Benutzung findet nur eine minimale Relativbewegung des Prothesenschafts 1 zum Extremitätenstumpf E statt.

Die Exoprothese 3 in unterschiedlichen Ausführungen, hier als Haken oder Kletterwerkzeug ausgeführt, wird durch Verbindungsmittel 30 an der Kupplung 2 auswechselbar befestigt. Damit kann ein schneller Austausch von Exoprothesen 3 erreicht werden. Die Exoprothese 3 kann aber auch an die Kupplung 2 angeformt sein bzw. die Kupplung 2 Teil der Exoprothese 3 sein.

Als Verbindungsmittel 30 kann eine Schlaufe verwendet werden, die an der Exoprothese 3 befestigt ist und eine direkte Anbindung zum Seil 1121 hat. Über das Seil 1121 und die Verschlusseinrichtung 112 kann eine Relativbewegung zwischen Prothesenschaft 1 und Exoprothese 3 erreicht werden, wobei der Einschnüreffekt ebenfalls auftritt.

Zusätzlich zur optimierten Anpassung wird ein verbesserter Sitz der Prothese 0 beim Klettern und/oder Bouldern erreicht.

Die Kupplung 2 weist eine Kupplungsaussenfläche 20 auf, an welcher eine Mehrzahl von Kupplungshaltemitteln 21 verteilt angeordnet sind. Die Kupplungshaltemittel 21 halten das mindestens eine Spannmittel 1121, welches hier von der Schalenaussenfläche 111 der Aussenschale 11 über einen Spalt zwischen Prothesenschaft 1 und Kupplung 2 geführt verläuft, ebenfalls. Von der Schalenaussenfläche 111 ist mindestens ein Spannmittel 1121 bis zu mindestens einem Kupplungshaltemittel 21 geführt und wird dort umgelenkt. Die Kupplungshaltemittel 21 können auch in Form von Umlenkrollen ausgebildet sein.

Das dem distalen Ende des Prothesenschafts 1 zugewandte proximale Ende der Kupplung 2 und das distale Ende des Prothesenschafts 1 sind derart gestaltet, dass in einem ausgelenkten Zustand der Prothese 0 gemäss Figur 2 eine Relativbewegung mit einer Auslenkung ΔI erreichbar ist. Die Relativbewegung zwischen Prothesenschaft 1 und Kupplung 2 und damit der Exoprothese 3 ist mit einem Doppelpfeil kenntlich gemacht. Durch das Spannmittel 1121 zwischen Halte- und Umlenkmitte 1122 und Kupplungshaltemitteln 21 wird die lineare Relativbewegung zwischen Prothesenschaft 1 und Kupplung 2 gehemmt.

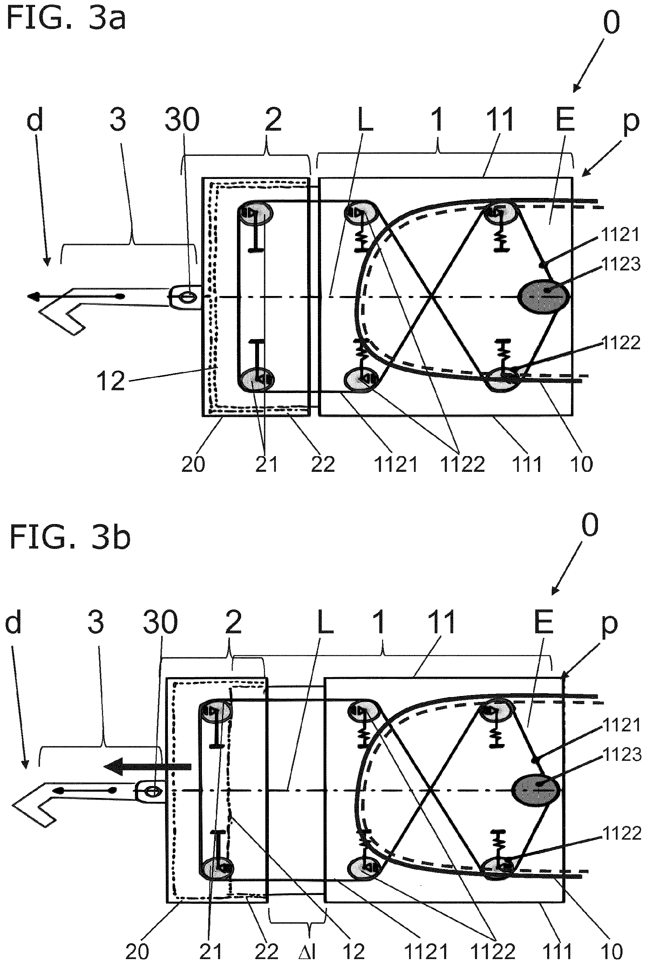

In der schematischen Darstellung gemäss Figur 3a ist eine Prothese 0 gezeigt, wobei das distale Ende des Prothesenschafts 1 in das proximale Ende der Kupplung 2 in axialer Richtung eingeführt gelagert ist.

Am distalen Ende des Prothesenschafts 1 ist hier eine Prothesenschaftführung 12 angeordnet, welche in ein Kupplungsführungsmittel 22 in der Kupplung 2, welches beispielsweise als Aussparung 22 ausgeführt sein kann, eingeführt gelagert ist. Die Kupplung 2 umgibt hier die Prothesenschaftführung 12 mindestens teilweise hülsenartig. Die Prothesenschaftführung 12 ist teilweise in die Aussparung 22 eingesteckt geführt gelagert. Figur 3a zeigt den Grundzustand der Prothese 0, wobei ein minimaler Spalt bzw. Abstand zwischen Kupplung 2 und Prothesenschaft 1 besteht.

Die Verbindung zwischen Prothesenschaft 1 und der Kupplung 2 ist durch die Spannmittel 1121 erreicht, welche an den Halte- und Umlenkmitteln 1122 auf der Schalenaussenfläche 111 und Kupplungshaltemitteln 21 auf der Kupplungsaussenfläche 20 geführt gehalten sind. Durch die Prothesenschaftführung 12, die in die Aussparung 22 in der Kupplung 2 geführt ist, wird eine geführte lineare Relativbewegung zwischen Prothesenschaft 1 und Kupplung 2 erreicht. Durch die Spannmittel 1121 und die Halte- und Umlenkmitteln 1122 ist diese Relativbewegung definierbar, je nach Krafteinwirkung von einem nicht ausgelenkten Grundzustand bis in ausgelenkte Zustände.

In Figur 3b ist eine Prothese 0 unter Zugspannung dargestellt, wobei der Prothesenschaft 1 relativ zur Kupplung 2 und damit zur Exoprothese 3 ausgelenkt ist. Durch eine Zugkraft entsteht eine Zugspannung in Form einer Normalspannung senkrecht zur distalen Endfläche der Kupplung 2 bzw. des Prothesenschafts 1. Aufgrund des Flaschenzugprinzips wird hier die Zugkraft, welche von der Exoprothese 3 über die Kupplung 2 auf den Prothesenschaft 1 wirkt, durch das umgelenkte Seil auf mehrere Punkte flächig verteilt, welche über die Schalenaussenfläche 111 und die Kupplungsaussenfläche 20 verteilt sind. Da eine lineare Relativbewegung des Prothesenschafts 1 zur Kupplung 2 entlang der Längsachse L möglich ist, bildet sich je nach wirkender Zugkraft bzw. Zugspannung ein Spalt mit einer Grösse ΔI aus. Während die Verschlusseinrichtung 112, welche entlang der Kupplung 2 und Prothesenschaft 1 verteilt ist die Zugspannung aufnimmt, taucht die Prothesenschaftführung 12 mehr oder weniger tief in die Aussparung 22 der Kupplung 2 ein bzw. wird herausgezogen.

Die Verschlusseinrichtung 112 hält über den Spalt hinweg, die Auslenkung des Prothesenschafts 1 relativ zur Kupplung 2. Da die Zugkraft nur indirekt und flächig verteilt auf den Prothesenschaft 1 wirkt und durch die Verschlusseinrichtung 112 als Seilzugsystem teilweise abgefangen wird, resultiert ein verbessertes Tragegefühl der Prothese 0. Da Zugkräfte vermehrt beim Klettern und Bouldern wirken, ist diese Prothese 0 prädestiniert für den Einsatz im Sport.

In einer einfachsten Ausgestaltung sollte die Aussparung 22 in der Kupplung 2 und die Prothesenschaftführung 12 zylindrisch ausgestaltet sein. Die zylindrische Oberfläche der Prothesenschaftführung 12 kann dann ohne Weiteres mit der Innenwand der Aussparung 22 wirkverbunden werden, also geführt anliegen. Die Querschnittsflächen der Aussparung 22 in der Kupplung 2, einer Aussparung in der Exoprothese 3 und der Prothesenschaftführung 12 können aber auch Vieleckig ausgeführt werden, insbesondere sechseckig oder achteckig. Damit wäre eine Verdrehsicherung ohne Weiteres geschaffen.

Um auch noch eine Verdrehsicherung der Prothesenschaftführung 12 in der Aussparung 22 zu erreichen, können entlang der Oberfläche der Prothesenschaftführung 12 und der Innenwand der Aussparung 22 Nuten oder Sicken angeordnet werden. Diese sind hier nicht dargestellt. Um ein ungewünschtes Herausrutschen der Prothesenschaftführung 12 aus der Aussparung 22 bei zu hohen Zugkräften zu verhindern, kann entlang der Aussparung 22 und/oder der Prothesenschaftführung 12 ein Rückhaltemittel, beispielsweise in Form eines Anschlages angeordnet sein. Ein solcher Anschlag könnte ein abgekröpftes Ende 200 der Kupplungsaussenfläche 20 und eine mindestens teilweise umlaufende Wulst 120 am Ende der Prothesenschaftführung 12 umfassen. Dadurch kann der Prothesenschaft 1 nicht weiter nach rechts von der Kupplung 2 beabstandet werden und eine Maximalauslenkung des Prothesenschafts 1 in Richtung der Längsachse L relativ zur Kupplung 2 ist erreichbar.

Ebenfalls möglich ist die Ausgestaltung des distalen Endes des Prothesenschafts 1 als Hülse mit einer Aussparung, in welche eine Führung am proximalen Ende der Kupplung 2 einführbar ist. Anstelle einer Prothesenschaftführung 12 wäre eine Kupplungsführung angeordnet, welche in eine Aussparung im Prothesenschaft 1 einführbar ist.

Diese Aussparung im Prothesenschaft 1 und die Kupplungsführung können wie oben erläutert bevorzugt zylindrisch ausgestaltet sein. Die zylindrische Oberfläche der Kupplungsführung kann dann ohne Weiteres mit der Innenwand der Aussparung im Prothesenschaft 1 wirkverbunden werden, also geführt anliegen. Auch hier kann eine Verdrehsicherung durch Nuten und/oder Sicken entlang der Oberfläche der Kupplungsführung und der Innenwand der Aussparung im Prothesenschaft 1 angeordnet, erreicht werden. Die oben beschriebenen Rückhaltemittel sind ebenfalls am distalen Ende des Prothesenschafts 1 und am proximalen Ende der Kupplung 2 analog anordbar.

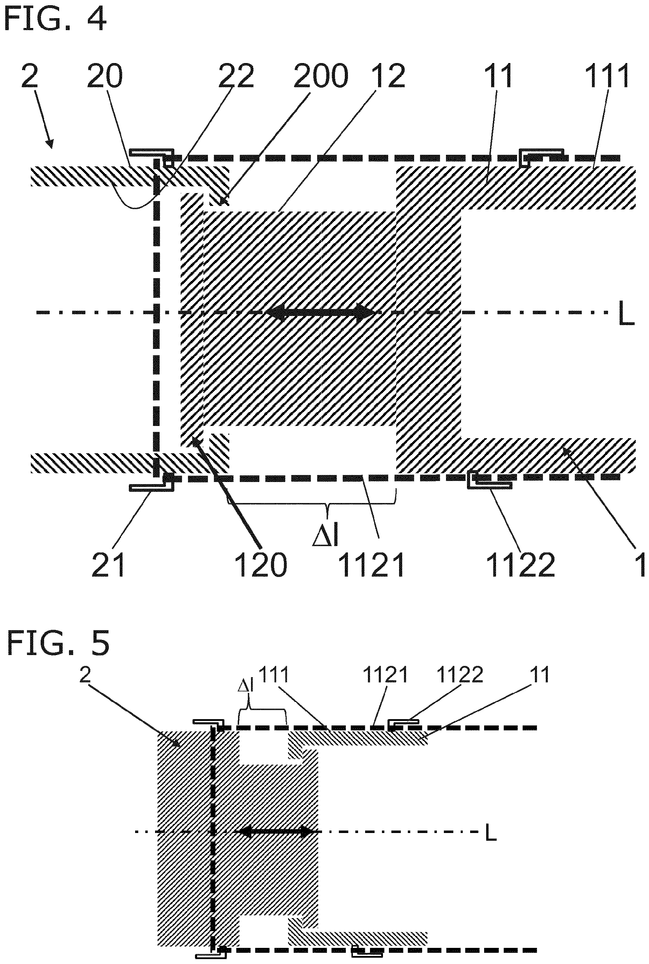

In Figur 5 ist eine abgewandelte Gestaltung gezeigt. Hier ist das proximale Ende der Kupplung 2 als Führung ausgeführt ist und in eine Aussparung im distalen Ende des Prothesenschafts 1, welche hülsenförmig ausgestaltet ist, eingeführt. Obige Merkmale für verbesserte Führungseigenschaften und Verdrehsicherung sind analog ausführbar.

In allen Ausführungsformen wird durch die lineare Relativbewegung der Kupplung 2 bei einer aufgebrachten Zugkraft an der Exoprothese 3 eine zusätzliche Zugspannung in das Spannmitteln 1121 eingebracht, welche eine zusätzliche umschliessende Kraft auf den Prothesenschaft 1 und damit auf den Prothesenschuh 10 und den Extremitätenstumpf E verursacht. Aufgrund dieser Funktion ist eine erhöhte Zugbelastung möglich ist. Die maximale Zugbelastbarkeit ist proportional zur Zugbelastung/ aufgebrachten Zugkraft. Aufgrund der beabstandbaren Ausbildung von Kupplung 2 zum Prothesenschaft 1 variiert je nach Zugbelastung die Umspannung des Extremitätenstumpfes E durch den Prothesenschaft 1.

Die Verschlusseinrichtung 112 hat immer zwei Funktionen. Zum einen ist die Einschnürung der Aussenschale 11 etwa senkrecht zur Längsachse L erreicht, welche durch durch den Verschluss 1123 festgestellt wird. Zum zweiten wird durch die Verbindung der Spannmittel 1121 mit den Kupplungshaltemitteln 21 auch eine lineare Relativbewegung in Richtung Längsachse L gehemmt.

Die Spannmittel 1121, die als mindestens ein Seil 1121 ausgeführt sein können, sind beispielsweise aus Polyamid, Polyethylen, einem Monofilamentpolymer, aus Dyneema® (Ultra-High-Molecular-Weight Polyethylene) oder aus einem ummantelten Stahlseil gebildet. Als Ummantelung können verschiedene Kunststoffe gewählt werden unter anderem Polyamid. Das mindestens eine Seil 1121 ist extrem strapazierfähig, schmutzabweisend und leicht zu reinigen. Üblicherweise hat das Seil 1121 einen Durchmesser zwischen 0.5 mm und 1.5 mm. Das Seil 1121 kann aus einer Faser oder einer Mehrzahl von miteinander verbundenen Fasersträngen aufweisen. Die Faserstränge können beispielsweise miteinander verwebt sein.

Die Aussenschale 11, die Halte- und Umlenkmittel 1122 der Verschluss 1123 und die Kupplung 2 müssen widerstandsfähig sein und möglichst leicht. Als mögliche Materialien kommen verschiedene Kunststoffe in reiner oder faserverstärkter Form, beispielsweise Polyethylen oder Polypropylen in verschiedenen Dichten, in Frage. Bevorzugt wird eine Polypropylenlage mit Polypropylenfasern verstärkt eingesetzt, ein Material, welches unter dem Handelsnamen Curv® von der Firma Propex Fabrics erhältlich ist und beispielsweise in der Herstellung von Koffern genutzt wird. Auch PE12 (=Nylon 12) ist ein bevorzugtes Material.

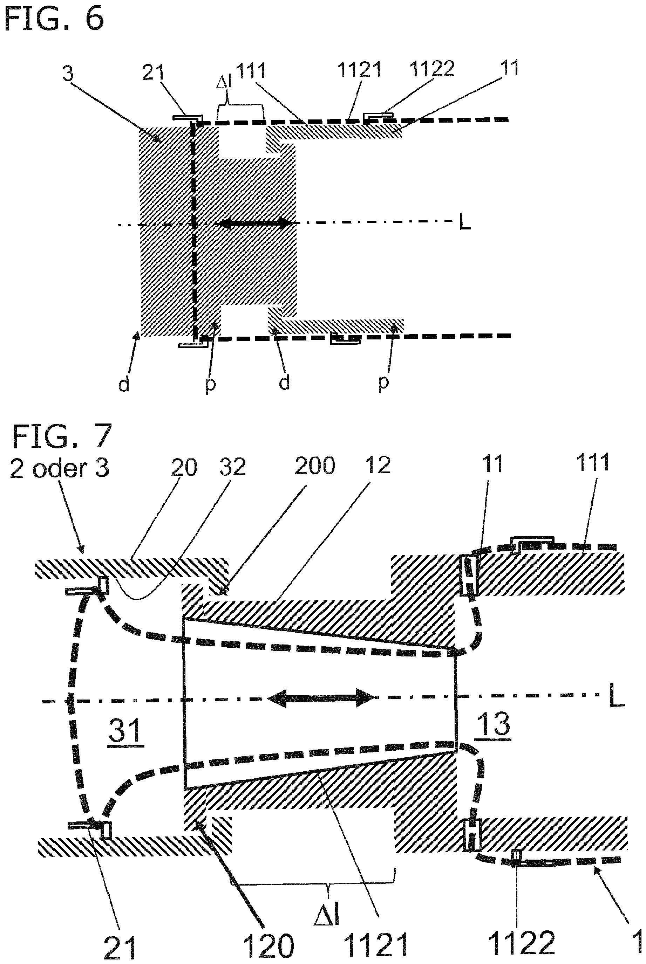

Wie in Figur 6 dargestellt, kann die Kupplung 2 an die Exoprothese 3 auch angeformt oder in diese integriert ausgestaltet sein. Damit wäre nur noch ein kombiniertes Bauteil 2, 3 vorhanden. Der Prothesenschaft 1 bleibt mit der Aussenschale 11, der Aussenschale 111 und der Verschlusseinrichtung 112, wie oben erläutert gleich. Die Spannmittel 1121 verlaufen entsprechend von der Schalenaussenfläche 111 von Halte- und Umlenkmitteln 1122 bis zu Kupplungshaltemitteln 21. Die Kupplungshaltemittel 21 sind dabei an der Aussenfläche der Exoprothese 3, am proximalen Ende der Exoprothese 3, angeordnet. Bei Zugbelastung bildet sich der Spalt ΔI zwischen proximalem Ende p der Exoprothese 3 und distalem Ende d des Prothesenschafts 1.

In einer leicht abgewandelten Bauform, sind Kupplung 2 und Exoprothese 3 ebenfalls integriert und es ist ein Spalt ΔI zwischen Kupplung2/Exoprothese 3 und dem Prothesenschaft 1 bei Zugbelastung ausbildbar. Die Spannmittel 1121 werden entlang der Schalenaussenfläche 111 geführt, verlaufen dann teilweise durch einen Prothesenschaftinnenraum 13 und sind dann vom distalen Ende d des Prothesenschafts 1 zum proximalen Ende p der Kupplung 2 bzw. der Exoprothese 3 geführt, wobei die Spannmittel 1121 dort in einen Innenraum 31 in der Kupplung 2 bzw. der Exoprothese 3 geführt sind. Hier sind die Kupplungshaltemittel 21 an einer Innenfläche 32 des Innenraums 31 angeordnet und mit den Spannmitteln 1121 bis zur Schalenaussenfläche 111 bzw. den Halte- und Umlenkmitteln 1122 wirkverbunden. Der Spalt Δl wird auch hier bei Zugkraftbelastung auf die Exoprothese 3 zwischen Kupplung 2/Exoprothese 3 und dem Prothesenschaft 1 ausgebildet. Damit das mindestens eine Spannmittel 1121 in den Prothesenschaftinnenraum 13 geführt werden kann, sind hier zwei Durchgänge durch die Aussenschale 11 vorgesehen. Das Spannmittel 1121 muss durch die Kupplungshaltemittel 21 an der Aussenfläche der Kupplung 2 oder der Exoprothese 3 bzw. an der Innenfläche 32 der Exoprothese 3 oder der Kupplung 2 gefädelt werden, damit eine Wirkverbindung erreicht wird.

### Bezugszeichenliste

0 Prothese
1 Prothesenschaft
10 schlauchförmiger Prothesenschuh
11 Aussenschale (einschnürbare)
111 Schalenaussenfläche
112 Verschlusseinrichtung
   1121 Spannmittel/Seil
   1122 Halte- und Umlenkmittel/Haken
   1123 Verschluss
12 Prothesenschaftführung (bevorzugt zylindrisch, mit Nuten für Verdrehsicherheit)
120 mindestens teilweise umlaufende Wulst
13 Prothesenschaftinnenraum
2 Kupplung für Exoprothese
20 Kupplungsaussenfläche
   200 abgekröpftes Ende
21 Kupplungshaltemittel (Haken an Aussenfläche)
22 Kupplungsführungsmittel
   Rückhaltemittel (optional, verhindern ausfädeln der Kupplung aus Prothesenschaft)
3 Exoprothese/ Kletterwerkzeug/Werkzeug
30 Verbindungsmittel der Exoprothese
31 Innenraum
32 Innenfläche
E Extremitätenstumpf /Unterarmstumpf
p proximal (Körper zugewandt)
d distal
ΔI lineare Auslenkung
L Längsachse

## Patentansprüche

1. Prothese (0), umfassend einen Prothesenschaft (1) an dessen distalen Ende eine Exoprothese (3) angeordnet ist, wobei der Prothesenschaft (1) eine einschnürbare Aussenschale (11) mit einer Schalenaussenfläche (111) umfasst und eine Verschlusseinrichtung (112) mit mindestens einem Spannmittel (1121), Halte- und Umlenkmitteln (1122) und mindestens ein Verschluss (1123) flächig auf der Schalenaussenfläche (111) verteilt angeordnet ist,
**dadurch gekennzeichnet, dass**
der Prothesenschaft (1) mindestens annähernd linear in Richtung Längsachse (L) relativ zur Exoprothese (3) einen Spalt (ΔI) zwischen Prothesenschaft (1) und Exoprothese (3) ausbildend verschiebbar an der Exoprothese (3) oder verschiebbar an einer Kupplung (2) zwischen Prothesenschaft (1) und Exoprothese (3) lösbar befestigbar ist,
wobei das mindestens eine Spannmittel (1121) der Verschlusseinrichtung (112) mit einer Mehrzahl von Kupplungshaltemitteln (21) an der Kupplung (2) oder der Exoprothese (3) derart gespannt wirkverbindbar ist, dass im montierten Einsatzzustand je nach Zugbelastung auf die Exoprothese (3) die lineare Relativbewegung vom mindestens einen Spannmitteln (1121) entlang der Halte- und Umlenkmitteln (1122) an der Schalenaussenfläche (111) bis zu den Kupplungshaltemitteln (21) bis zu einem Maximalabstand gehemmt wird und die Einschnürung der Aussenschale (11) senkrecht zur Längsachse (L) transradial variierbar ist.

2. Prothese (0) nach Anspruch 1, wobei die Kupplungshaltemittel (21) an einer Kupplungsaussenfläche (20) der Kupplung (2) oder einer Aussenfläche der Exoprothese (3) angeordnet und mit dem mindestens einen Spannmitteln (1121) direkt wirkverbindbar sind.

3. Prothese (0) nach Anspruch 1, wobei die Kupplungshaltemittel (21) an einer Innenfläche (32) der Kupplung (2) bzw. im Innenraum der Kupplung (2) oder an einer Innenfläche (32) der Exoprothese (3) bzw. in einem Innenraum (31) der Exoprothese (3) angeordnet sind und mit dem mindestens einen Spannmittel (1121) nach Durchgang durch einen Prothesenschaftinnenraum (13) und den Innenraum (31) der Exoprothese (3) wirkverbindbar sind.

4. Prothese (0) nach einem der vorhergehenden Ansprüche, wobei die Kupplung (2) an der Exoprothese (3) angeformt ist.

5. Prothese (0) nach einem der vorhergehenden Ansprüche, wobei das distale Ende des Prothesenschafts (1) als Prothesenschaftführung (12) ausgeführt ist und in ein als Aussparung ausgestaltetes Kupplungsführungsmittel (22) im proximalen Ende der Kupplung (2) oder im proximalen Ende der Exoprothese (3), welches teilweise hülsenförmig ausgestaltet ist, eingeführt ist.

6. Prothese (0) nach einem der vorhergehenden Ansprüche, wobei das proximale Ende der Kupplung (2) oder der Exoprothese (3) als Führung ausgeführt ist und in eine Aussparung im distalen Ende des Prothesenschafts (1), wobei die Aussparung teilweise hülsenförmig ausgestaltet ist, eingeführt ist.

7. Prothese (0) nach einem der Ansprüche 1 bis 5, wobei die Aussparung (22) in der Kupplung (2) oder der Exoprothese (3), die Prothesenschaftführung (12) oder die Aussparung im distalen Ende des Prothesenschafts (1) und die Führung am proximalen Ende der Kupplung (2) oder der Exoprothese (3) bevorzugt jeweils zylindrisch ausgestaltet sind und damit Zylinderoberflächen aufweisen oder vieleckig, insbesondere sechseckig oder achteckig ausgeführt sind.

8. Prothese (0) nach einem der Ansprüche 5 bis 7, wobei entlang der Oberflächen der Prothesenschaftführung (12), der Aussparung (22) in der Kupplung (2) oder der Exoprothese (3), der Aussparung im distalen Ende des Prothesenschafts (1) und/oder der Führung am proximalen Ende der Kupplung (2) oder der Exoprothese (3) Nuten oder Sicken angeordnet sind.

9. Prothese (0) nach einem der Ansprüche 5 bis 8, wobei entlang der Aussparung (22) und der Prothesenschaftführung (12) bzw. entlang der Aussparung im distalen Ende des Prothesenschafts (1) und der Führung am proximalen Ende der Kupplung (2) oder der Exoprothese (3) Rückhaltemittel angeordnet sind.

10. Prothese (0) nach Anspruch 9, wobei, als Rückhaltemittel das Ende der Kupplungsaussenfläche 20 oder der Aussenfläche der Exoprothese (3) als mindestens teilweise abgekröpftes Ende 200 und das distale Ende der Prothesenschaftführung (12) als mindestens teilweise umlaufende Wulst (120) ausgebildet ist, sodass ein Anschlag erreicht ist.

11. Prothese (0) nach Anspruch 9, wobei, als Rückhaltemittel das Ende der Kupplungsaussenfläche 20 oder der Aussenfläche der Exoprothese (3) als mindestens teilweise umlaufende Wulst und das distale Ende der Prothesenschaftführung (12) als mindestens teilweise abgekröpftes Ende ausgebildet ist, sodass ein Anschlag erreicht ist.

12. Kupplung (2) einer Prothese (0), zur Ankupplung einer Exoprothese (3) an einen Prothesenschaft (1), wobei der Prothesenschaft (1) eine Aussenschale (11) mit einer Schalenaussenfläche (111) aufweist, auf welcher flächig verteilt eine Verschlusseinrichtung (112) mit mindestens einem Spannmittel (1121), mehreren Halte- und Umlenkmitteln (1122) und einem Verschluss (1123) angeordnet ist,
**dadurch gekennzeichnet, dass**
die Kupplung (2) eine Kupplungsaussenfläche (2) aufweist, auf welcher verteilt eine Mehrzahl von Kupplungshaltemitteln (21) verteilt ist, wobei die Kupplungshaltemittel (21) mit dem mindestens einen Spannmittel (1121) der Verschlusseinrichtung (112) über die Kupplungsaussenfläche (2) und die Schalenaussenfläche (111) geführt wirkverbindbar sind, sodass eine lineare Relativbewegung der Kupplung (2) zum Prothesenschaft (1) in Richtung Längsachse (L) erreicht ist.

13. Kupplung (2) nach Anspruch 12, wobei ein als Aussparung ausgestaltetes Kupplungsführungsmittel (22) im proximalen Ende der Kupplung (2), welches teilweise hülsenförmig ausgestaltet ist, angeordnet ist.

14. Kupplung (2) nach Anspruch 13, wobei das proximale Ende der Kupplung (2) als Führung ausgeführt ist.

15. Kupplung (2) nach einem der Ansprüche 13 bis 14, wobei entlang der Oberfläche der Aussparung (22) in der Kupplung (2) Nuten oder Sicken angeordnet sind.
